# EUROPEAN PATENT APPLICATION

(11) **EP 0 526 093 A1**
(43) Date of publication of application: **03.02.1993**
(21) Application number: 92306717.7
(22) Date of filing: 23.07.1992
(51) Int. Cl.: A61K 31/19, A61K 31/16, A61K 31/21, C07C 233/25, C07C 323/52, C07F 9/38, C07C 381/02, C07D 213/75, A61K 31/66, A61K 31/44

(54) **New non-steroidal agents as 5-alpha reductase inhibitors**

(30) Priority: 29.07.1991 US 737097
(71) Applicant: MERCK & CO. INC., Rahway New Jersey 07065-0900 (US)
(72) Inventor: Witzel, Bruce E., Westfield, NJ 07090 (US); Tolman, Richard L., Warren, NJ 07059 (US)
(74) Representative: Thompson, John Dr.

(57) **Abstract**

Described are new non-steroidal drugs for treatment of benign prostatic hyperplasia and other disorders mediated by high 5-alpha reductase activity, high dihydrotestosterone levels, and other conditions of hyperandrogenic stimulation, of the formula: 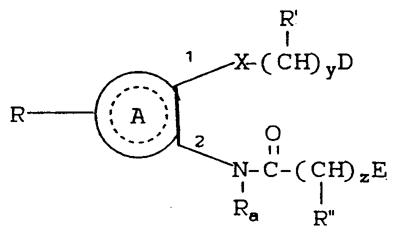
wherein
A, D, E, X, R, R′, R˝, Rₐ, y and z are as defined in claim 1.

## Description

### BACKGROUND OF THE INVENTION

It is well known in the art that certain undesirable physiological manifestations, such as acne vulgaris, seborrhea, female hirsutism, and male pattern baldness and particularly benign prostatic hypertrophy (BPH), art the result of hyperandrogenic stimulation caused by an excessive accumulation of testosterone or similar androgenic hormones in the metabolic system. Early attempts to provide a chemtherapeutic agent to counter the undesirable results of hyperandrogenicity resulted in the discovery of several steroidal antiandrogens having undesirable hormonal activities of their own.

It more recently became known in the art that the principal mediator of androgenic activity in some target organs is 5α-dihydrotestosterone, and that it is formed locally in the target organ by the action of testosterone-5α-reductase or other 5α-reductase isozymes. It is known that various members of the 5α-reductase family of enzymes function to provide DHT levels in the control of various peripheral functions, i.e. male pattern baldness, beard growth, sebum production, etc. Any or all of these isozymic targets could be targets for a 5α-reductase inhibitor. It therefore has been postulated and demonstrated that inhibitors of testosterone-5α-reductase will serve to prevent or lessen symptoms of hyperandrogenic stimulation. Nayfe et al., Steriods, 14, 269 (1969) demonstrated in vitro that methyl 4-androsten-3-one-17β-carboxylate was a testosterone-5α-reductase inhibitor. Then Voigt and Hsia, Endocrinology, 92, 1216 (1973), Canadian Pat. No. 970,692, demonstrated that the above ester and the parent free acid, 4-androsten-3-one-17β-carboxylic acid are both active inhibitors of testosterone-5α-reductase in vitro. They further demonstrated that topical application of either testosterone or 5α-dihydrotesterone caused enlargement of the female hamster flank organ, an androgen dependent sebaceous structure. However, concomitant administration of 4-androsten-3-one-17β-carboxylic acid or its methyl ester inhibited the response elicited by testosterone but did not inhibit the respone elicited by 5α-dihydrotestosterone. These results were interpreted as indicating that the compounds were antiandrogenic by virtue of their ability to inhibit testosterone-5α-reductase.

Steroidal compounds particularly for the treatment of Benign Prostatic Hyperplasia (BPH) are well known in the art and constantly being developed. See the following patents: USP 4,317,817, USP 4,882,319 and EPO Publication Nos. 0 277 002, 0 343 954, 0 375 344, 0 375 345, 0 375 347, 0 375 349 (all assigned to SmithKline Beckmann Corporation) which disclose steroidal 5-alpha reductase inhibitors as BPH agents.

However, as described above, being steroids, they also can exhibit hormonal activity which may, in some cases, be undesirable, e.g. causing femininizing characteristics in men due to competition with testosterone or dihydrotesterone (DHT) for the androgen receptor.

Recent non-steroidal BPH active compounds have been disclosed in EP 291 245, EP 291 247 and EP 0 173 516 to ONO Pharmaceutical Co. However, these references do not describe o-substituted benzene derivatives which are dicarboxylates containing a carboxyalkyl amide chain, which is derived from an aliphatic carboxylate precursor.

It is highly desired in the art to discover new non-steroidal BPH agents which do not possess steroidal activity with their attendant undesirable hormonal effects in humans.

### SUMMARY OF THE INVENTION

By this invention is provided a new agent for the treatment of BPH of the following formula: 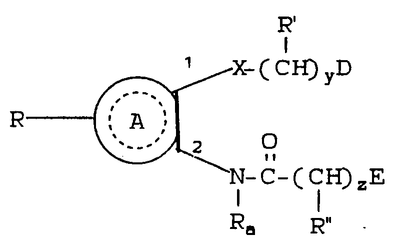
wherein
A is an 1,2-disubstituted aromatic ring selected from
(a) benzene, 1,2-disubstituted naphthalene;
(b) 5-6 membered heteroaromatic ring, containing 1-2 N atoms, 1 S or O atom, or combination thereof;

D and E are independently -COOH, -CONH₂, CONHR_{b}, COOR_{b}, SO₂OH, SO₃(OH), SO₂NH₂, -SSO₂ONa, PH(O)(OH), P(O)(OH)₂;
X is O, S, SO or SO₂;
R is H,
C₁-C₄ alkyl,
phenyl or substituted phenyl,
halo,
haloalkyl,
hydroxy,
carboxy,
cyano,
C₁-C₄ alkoxy,
C₁-C₄ alkylthio,
C₁-C₄ alkylsulfinyl,
C₁-C₄ alkylsulfonyl,
nitro,
amino,
C₁-C₄ mono or di-alkylamino;
R′ and R˝ are independently
H,
halo,
C₁-C₄ alkyl or C₁-C₄ alkoxy,
amino, or oxo, where CH-R′ or CH-R˝
in the formula become -C=O;
Rₐ is H, C₁-C₄ alkyl;
R_{b} is C₁-C₁₂ alkyl, phenyl or phenyl C₁-C₄ alkyl;
y is 1-6;
z is 6-20; and
wherein 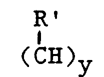
and 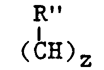
can independently represent substituted or unsubstituted alkyl radicals or alkenyl radicals containing at least one alkene bond;
and pharmaceutically acceptable salts and esters thereof.

The compounds of the instant invention are inhibitors of the human testosterone-5α-reductase .

### BRIEF DESCRIPTION OF THE INVENTION AND PREFERRED EMBODIMENTS

The scope of the compounds of the instant invention are described by the above-described formula.

In the description of the formula the following terms are used which are hereby defined:
X is preferably O or S, and particularly preferred is O.

"C₁-C₄ alkyl" includes linear or branched species, e.g. methyl, ethyl, n-propyl, isopropyl, cyclopropyl, n-butyl, isobutyl, sec-butyl, t-butyl and "C₁-C₁₂ alkyl" includes, alkyl up to 12 cartons including n-octyl, t-decyl, n-dodecyl.

"Phenyl C₁-C₄ alkyl" includes benzyl, 2-phenethyl and the like.

"C₁-C₄ alkoxy" includes linear or branched species, e.g., methoxy, ethoxy, n-propoxy, isopropoxy, n-butoxy, isobutoxy, sec-butoxy, t-butoxy.

"Halo" includes fluoro, chloro, bromo or iodo.

By the term "heteroaromatic ring" as used herein is meant a 5-6 membered ring, containing 1-2 N-atoms, 1 S or O atom, or combination thereof, and includes: pyridine, thiophene, furan, imidazole, 1,3-thiazole, 1,3-oxazole, 1,2,3-thiadiazole, and the like. The limitation here being that the 1,2-disubstitution occurs on only ring carbons of the heteroaromatic ring. Preferred heteroaromatic rings are pyridine, furan and thiophene.

"Substituted phenyl" includes phenyl substituted by one or more of C₁-C₄ alkyl, C₁-C₄ alkoxy, or halo, and the like, as defined above; representative examples include o, m-, p-methoxy phenyl; 2,4-dimethoxyphenyl; 2-chloro-4-ethoxyphenyl; 3,5-dimethoxyphenyl; 2,4-dichlorophenyl; 2-bromo-4-methylphenyl, o-fluorophenyl, and the like.

"Haloalkyl" includes C₁-C₄ alkyl, defined above, substitued with one or more "halo" as defined above and inlcudes: trifluoromethyl, 2,2-dichloroethyl and the like.

"C₁-C₄ alkylthio" includes C₁-C₄ alkyl, defined above, substituted with at least one divalent thio (-S-) grouping including; methylthio, ethylthio, isopropylthio, n-butylthio, and the like.

"C₁-C₄ alkylsulfinyl" includes C₁-C₄ alkyl, defined above, substituted with at least one -SO- grouping including; methylsulfinyl, ethylsulfinyl; isopropylsulfinyl, and the like.

"C₁-C₄ alkylsulfonyl" includes C₁-C₄ alkyl, defined above, substituted with at least one sulfonyl group, -SO₂-, including; methylsulfonyl, ethylsulfonyl, isopropylsulfonyl, n-butylsulfonyl, and the like.

"C₁-C₄ mono or dialkyl amino" includes amino, substituted with one or more C₁-C₄ alkyl groups as defined hereinabove, including: methylamino, ethylamino, n-butylamino, t-butylamino, dimethylamino, diethylamino, methyl-t-butylamino, and the like.

The R group or groups on the benzene or heteroaromatic ring can be present initially in the process, e.g. phenyl, methyl, methoxy, cyano carbomethoxy, trifluoromethyl, ( as in the starting o-nitrophenol 1 in Flow Chart A) or added later by a conventional reaction, e.g. chloro, as by chlorination, nitro by nitration, or created from a starting or added functional group present, e.g. converting a later added nitro group to an amino group by catalytic reduction, then alkylating to a mono or dialkylamine. An amino group can be subjected to diazotization to a hydroxy group, which can be followed by methylation to a methoxy group. Similarly, a hydroxy group can be converted to a thiol by the analogous procedures described in J. Org. Chem. 31, pp 3980-3984 (1966) by Newman and Karnes, and J. Org. Chem. 31, pp 410 (1966) by Kwart, H. and Evans, E.S. The resulting thiol can be alkylated to alkylthio, which can be oxidized to the corresponding sulfoxide or sulfone. Preferred substituents are H, C₁-C₄ alkyl, C₁-C₄ alkoxy and phenyl. These reactions and sequences are conventional in the art and it will be obvious to one skilled in the art to modify the benzene ring to arrive at an R radical disclosed herein.

By the term "pharmaceutically acceptable salts and esters thereof" is meant, salts and esters of the acid groups in the final molecule which can be used as part of the human drug delivery system and include the salts: sodium, potassium, calcium, ammonium, substituted ammonium, quaternary ammonium, and esters: ethyl ester, aceturate, besylate, edetate, phenpropionate, acetate, pamoate, and esters which serve as "prodrug" formulations which will hydrolyze in the body at physiological pH's to regenerate the acid, including pivaloylates, e.g. pivoxetil and pivoxil, and Kanebo esters, and the like. 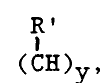
where y is 1-6, preferably 3, can contain at least one R′ substituent as defined above, and can be alkyl, e.g., -CH₂; -CH₂-CH₂; -CH₂-CH₂-CH₂, 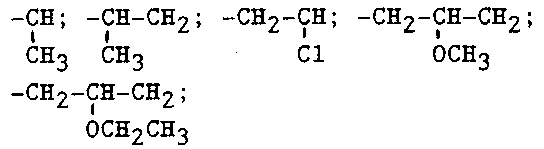
and the like.

An alkene bond can also be present in 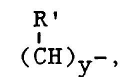
e.g., -CH₂-CH=CH;-CH₂-CH=CH-CH₂; -CH₂-CH₂-CH=CH; -(CH₂)₃ -CH=CH, and the like. 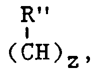
where z is 6-20, preferably 8-14, can contain at least one R˝ substituent as defined above, and can be alkyl; e.g., -(CH₂)₆- -(CH₂)₂₀; 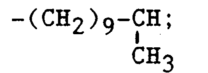 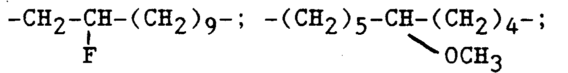
and the like.

An alkene bond can also be present in 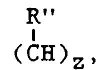
e.g.,
-CH₂-CH=CH-(CH₂)₈-; -(CH₂)₈-CH=CH-(CH₂)₂-; -(CH₂)₉-CH=CH-(CH₂)₉-; (CH₂)₄-CH=CH-(CH₂)₄-; and the like.

Preferred is where one R′ or R˝ is H and particularly preferred is where both 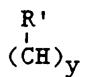
and 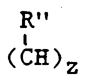
are alkyl.

Representative compounds of the instant invention within the above general formula are given by the following, structures;

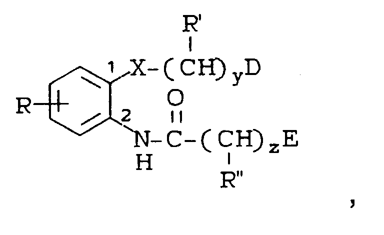

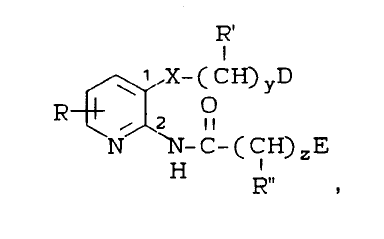

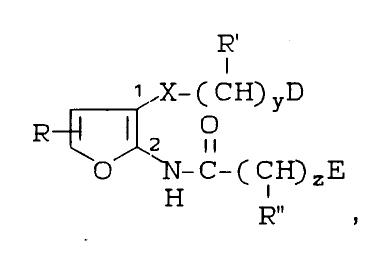

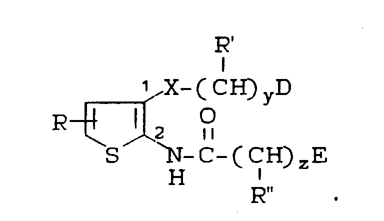

Particularly preferred are the following compounds: 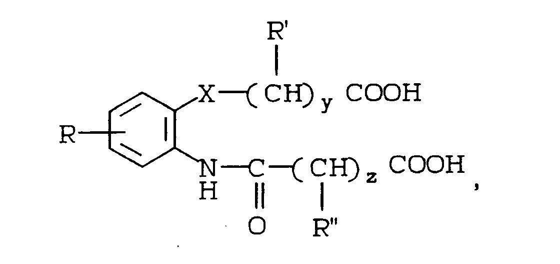 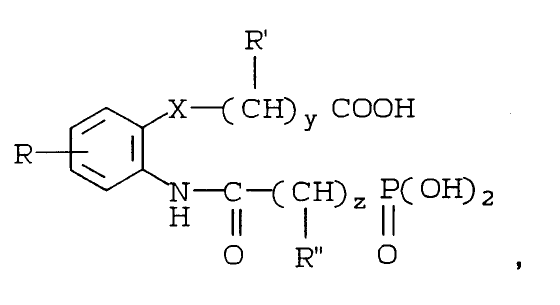 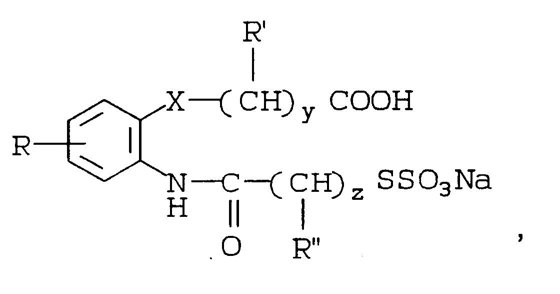 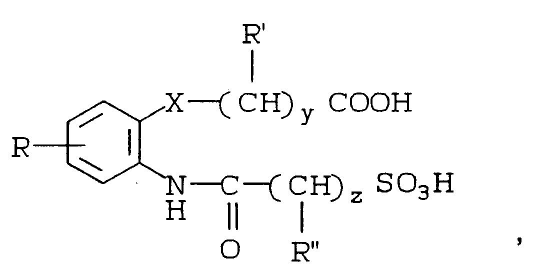 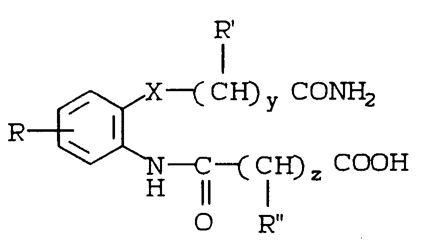
where X is O or S and R, R′, R˝, y and z are as defined above.

Preferred compounds within this class are: 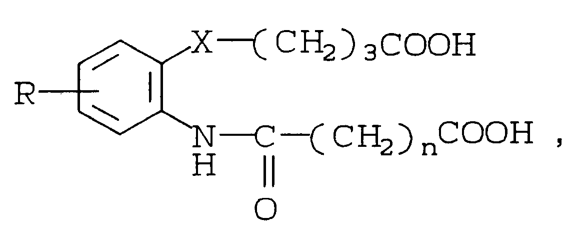 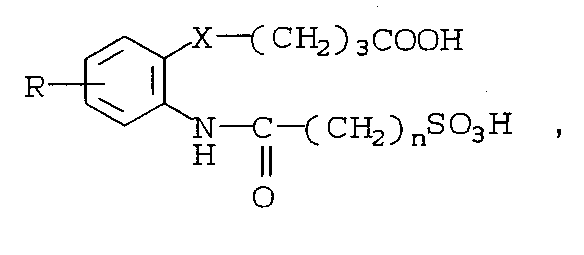 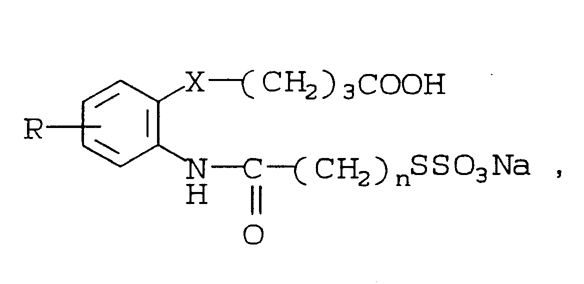 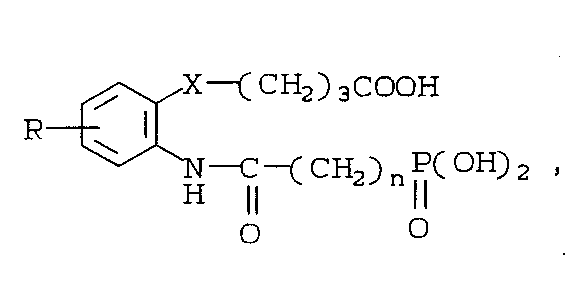 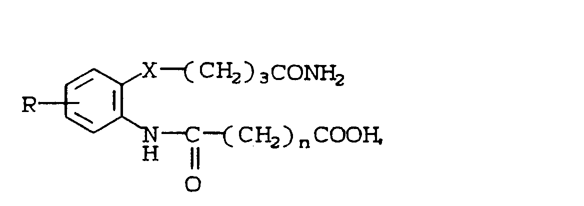
where X is O or S, and n is 8-14.

Also preferred compound are within these classes are: 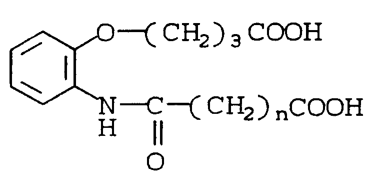
where n is 8-14. 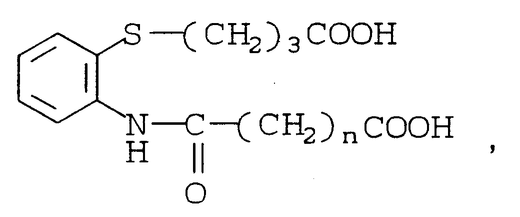
where n is 8-14.

The compounds of the instant invention can be made by the procedures outlined in the following Flowcharts.

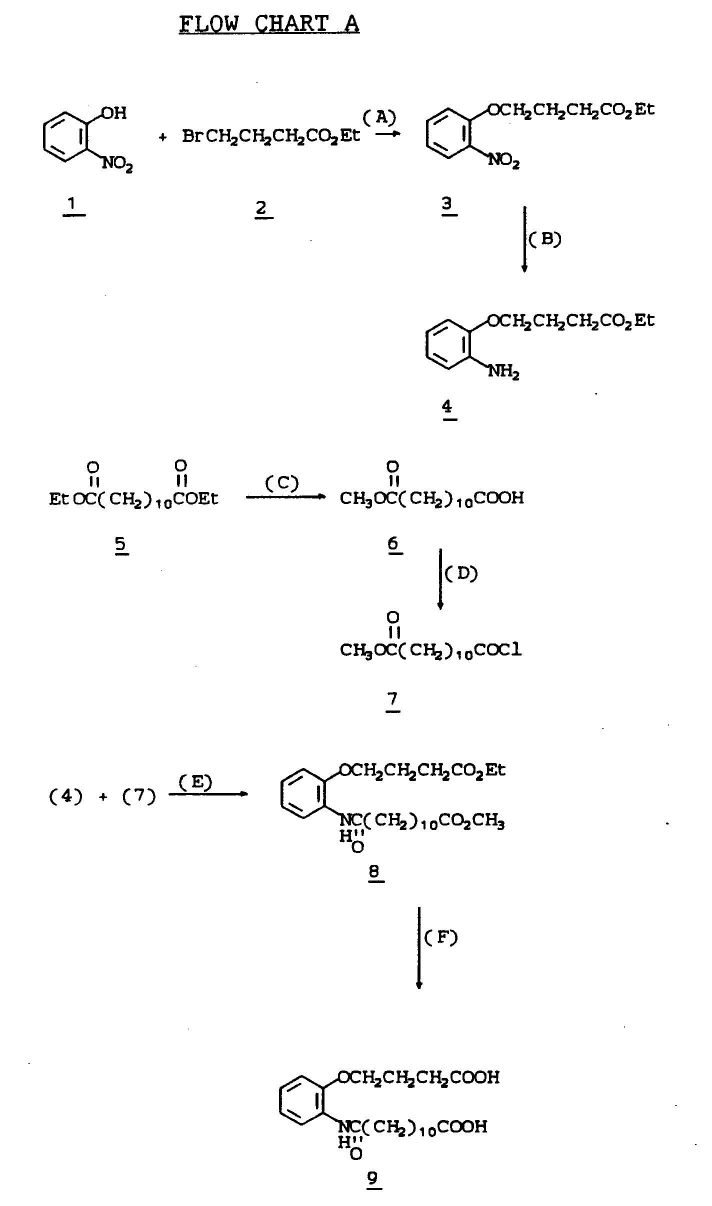

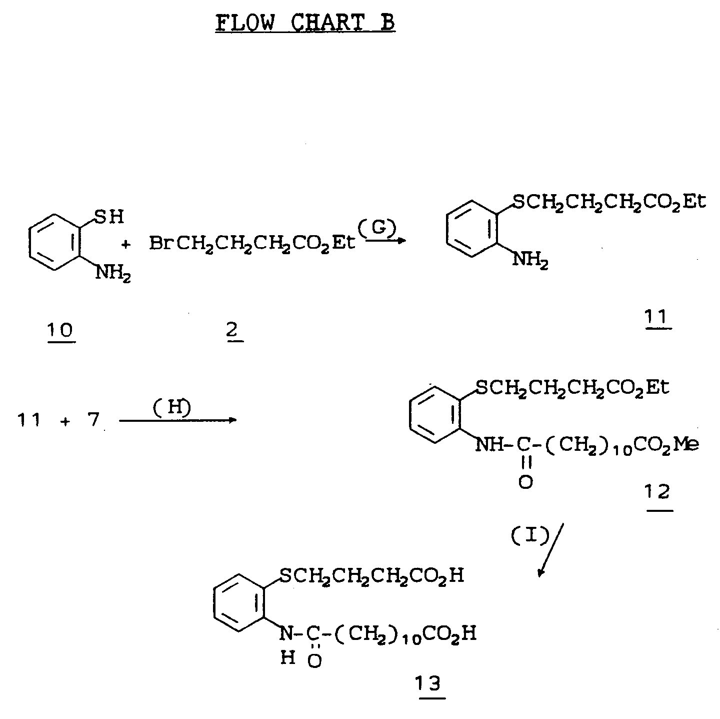

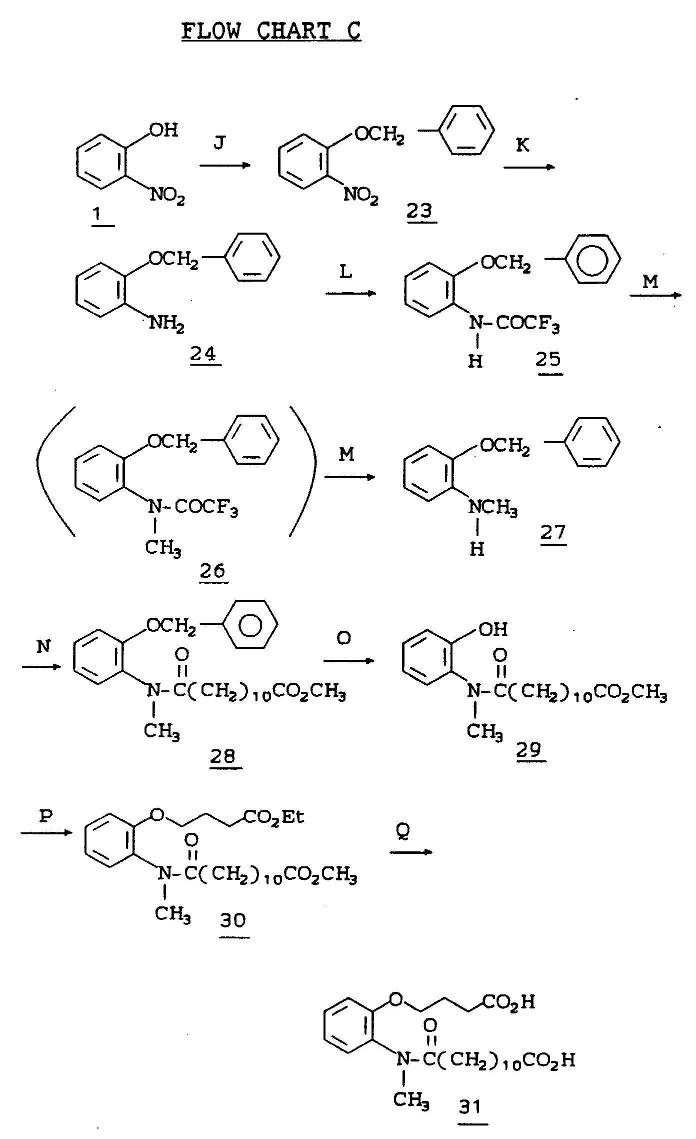

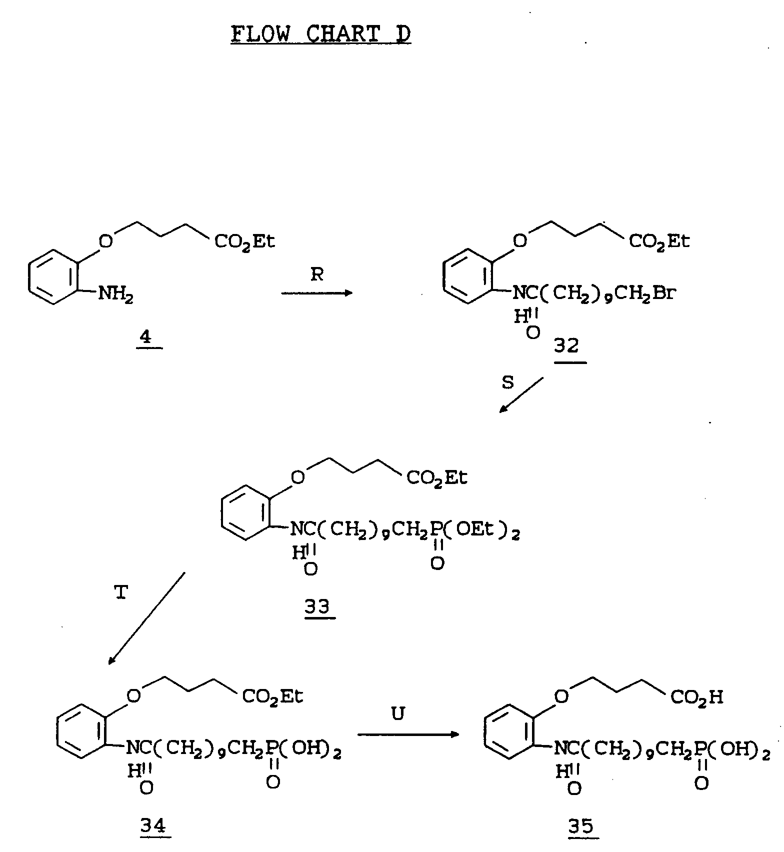

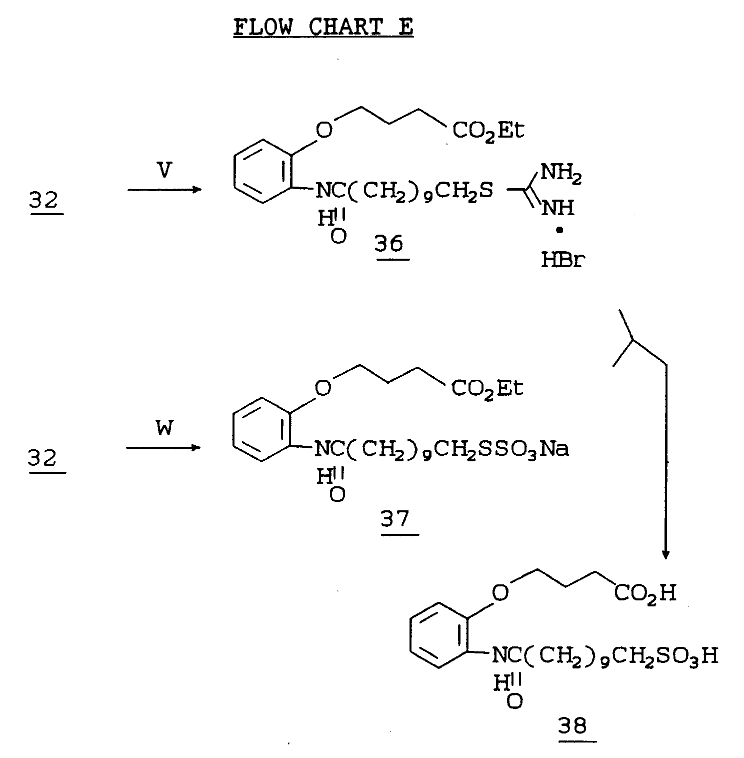

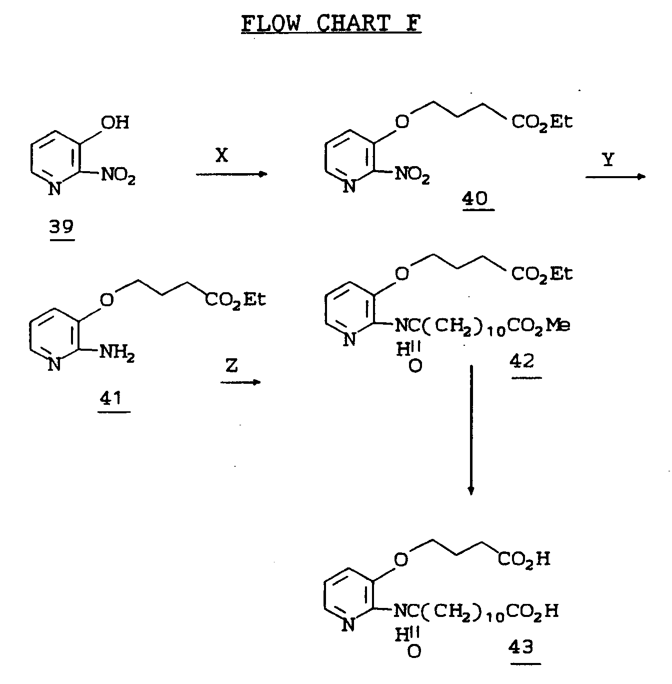

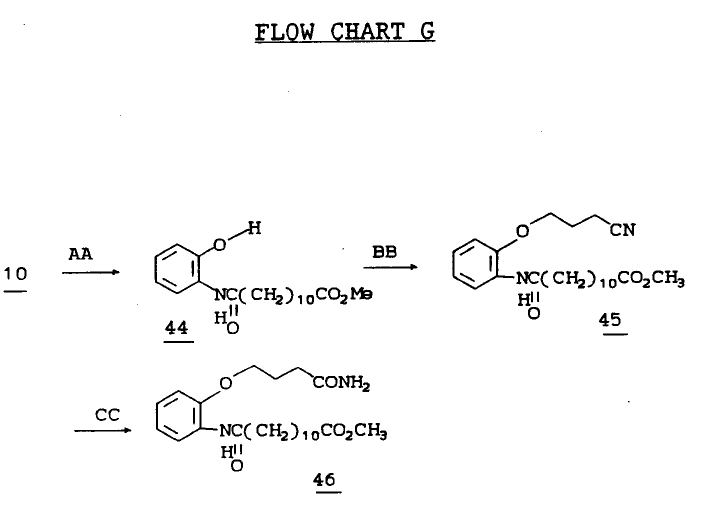

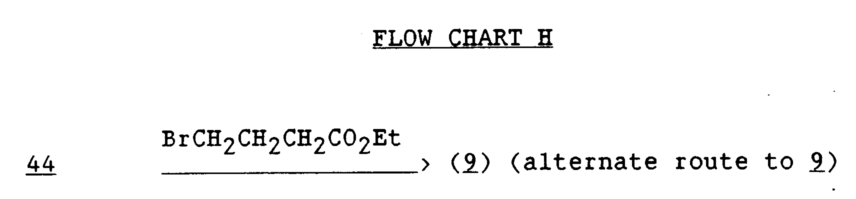

As seen in Flow Chart A, o-nitrophenol 1, ethyl 4-bromobutyrate 2 and anhydrous K₂CO₃ in e.g., dry acetone are heated at reflux or stirred for an extended period of time at room temperature, under a nitrogen atmosphere to product ethyl 4-(2-nitrophenoxy) butyrate 3, in Step (A).

A solution of 3 in e.g., ethyl acetate is catalytically hydrogenated at room temperature under e.g. 40 psig of H₂ in the presence of a 5% Pd/C catalyst to yield ethyl 4-(2-aminophenoxy)butyrate 4 in Step (B).

Step (C) comprises reacting diethyl dodecanoate 5 with barium hydroxide octahydrate in methanol at ambient temperature to obtain the monomethyl ester 6.

In Step (D) the mono ester mono acid 6 is refluxed with thionyl chloride for about 5 hours to produce the mono acid chloride, mono methyl ester 7.

Step (E) comprises the reaction of the mono acid chloride 7 with the amine 4 at e.g., 0-10°C in e.g., dry ether in the presence of a hydrogen acceptor e.g., triethylamine, to produce the amide 8.

In Step (F), the ether-amide diester 8 is de-esterified by e.g., 2.5 N NaOH in MeOH/H₂O to yield after acidification the final product, diacid 9.

Flow Chart B illustrates the synthesis of the corresponding thio compounds.

Step (G) illustrates the reaction of o-aminobenzenethiol with ethyl 4-bromobutyrate which can be carried out in e.g., dry dimethoxyethane, under dry N₂, in the presence of a proton acceptor, e.g., dry powdered K₂CO₃, to produce 11.

Step (H) illustrates the acylation of the amino group of 11 with ethyl 11-bromoundecanoate in a dry solvent, e.g., dry ether, at 0°C in the presence of an acid acceptor, e.g., pyridine.

Step (I) illustrates the hydrolysis of the diester to the final diacid 13, which can be accomplished with, e.g., NaOH/MeOH.

As seen in Flow Chart C, o-nitrophenol is benzylated under the same conditions as in Step (A).

Step (K) shows the reduction of the nitro group using e.g., Raney Ni in ethanol/NH₃ under 40 psig H₂.

Step (L) shows the trifluoroacetylation of 24 using e.g., trifluoroacetic anhydride in dry ether and powdered dry sodium carbonate.

Step (M) shows the N-methylation which can be accomplished using, e.g., methyl iodide in dry acetone and dry powdered KOH followed by removal of the N-trifluoroacetyl group with MeOH/H₂O.

Step (N) shows the N-acylation of 27, using an acid chloride, e.g., 7, in e.g., dry methylene chloride and pyridine at 0°C.

Step (O) shows the debenzylation of 28, which can be accomplished by e.g., 10% Pd/C in MeOH under a H₂ atmosphere.

Step (P) shows the O-alkylation of 29 using e.g., ethyl 4-bromobutyrate and K₂CO₃ in anhydrous acetone.

Step (Q) shows hydrolysis of the diester 30 to the diacid 31 by hydrolysis as e.g., described for Step (F).

Flow Chart D shows the production of phosphonate type esters and acids.

Step (R) shows the condensation of 4 with 11-bromoundecanoic acid in anhydrous methylene chloride using N,N′-dicyclohexylcarbodiimide and 4-dimethylaminopyridine to produce the important bromo intermediate 32.

Step (S) shows the reaction of 32 with triethylphosphite at e.g., 180°C under N₂ to produce the phosphonate ester 33.

Step (T) converts 33 via bromotrimethylsilane to the monoacid 34.

Step (U) uses the similar hydrolysis conditions of Step (F) to produce the phosphoniccarboxylic diacid 35.

The corresponding phosphinic acids can be analogously made from 32 by known procedures in the art.

Flow Chart E illustrates the synthesis of the sulfonic acid types of the invention compounds.

Step (V) shows reaction of the intermediate 32 with thiourea in EtOH/H₂O under N₂ at 90°C to yield the isothiouronium salt 36.

Step (W) shows the reaction of 32 with sodium thiosulfate under the conditions of Step (V) to yield the thiosulfate ester 37.

Further oxidation of esters 36 or 37 via the Showell or Ziegler procedures described in Step W in the examples yields the corresponding sulfonic acid 38.

The corresponding sulfinic acid can be prepared from 36 by the procedure of J. M. Sprague and T. B. Johnson, JACS 59, 2440 (1937).

The corresponding sulfonamide can be produced from the sulfonic acid 38, by protecting, e.g., the carboxylic acid via an ester, converting the sulfonic acid to a sulfonyl chloride, treating the sulfonyl chloride with ammonia and then hydrolyzing the protected carboxylic ester to the corresponding acid.

Flow Chart F shows the corresponding synthesis of the pyridine analogs of 8 and 9.

In Step (X), the nitrohydroxy pyridine is O-alkylated first using conditions analogous to Step (A) to produce 40.

Step (Y) shows reducing the nitro group in the same manner as described for Step (B).

Step (Z) shows acylating the amino group in the same manner as described for 8 in Step (E) to produce the diester 42. Hydrolysis produces the diacid 43.

Flow Chart G shows the production of some amides 46 of the invention.

In Step (AA), o-aminophenol is reacted directly with 7 under the conditions of Step (E) to produce the N-acylated phenol 44.

In Step (BB), O-alkylation of 44 as carried out using 4-bromobutyronitrile under conditions similar to Step (A), produces 45.

Step (CC) shows the hydrolysis of the nitrile to the amide 46 using MnO₂ in methylene chloride.

Flow Chart H illustrates an alternate route to 9 by starting with o-aminophenol, acylating to produce 44, then reacting 44 under the conditions of Step (A) to produce 8, then hydrolysis using Step (F) to yield 9.

Thus, by using the above described methods in the Flow Charts and the reaction starting materials and reagents described herein, all of the compounds described and encompassed by the claim can be synthesized by one skilled in the art.

It is obvious that other nitrophenols can be substituted for 1 in Flow Charts A and C to provide the scope of the compounds covered by this invention and include the following:
2-nitrophenol
2-nitro-6-methylphenol
2-nitro-5-methylphenol
2-nitro-4-methylphenol
2-nitro-3-methylphenol
2-nitro-4-phenylphenol
2-nitro-5-phenylphenol
2-nitro-4-chlorophenol
2-nitro-4-fluorophenol
2-nitro-4-trifluoromethylphenol
2-nitro-4-hydroxyphenol
2-nitro-4-methoxyphenol
2-nitro-6-ethoxyphenol
2-nitro-4-methylthio-phenol
2-nitro-4-methylsulfinylphenol
2-nitro-4-methylsulfonylphenol
4-nitro-3-hydroxypyridine
3-nitro-4-hydroxy-5-methylpyridine
3-nitro-4-hydroxy-6-methylpyridine
2-methyl-3-nitro-4-hydroxypyridine
2-hydroxy-3-nitro-5-phenylpyridine
2-nitro-3-hydroxy-5-phenylpyridine
2-hydroxy-3-nitro-5-chloropyridine
2-nitro-3-hydroxy-5-trifluoromethylpyridine
2-methoxy-4-nitro-5-hydroxypyridine
3-nitro-4-hydroxy-5-ethoxypyridine
2-methylthio-4-nitro-5-hydroxypyridine
2-nitro-3-hydroxy-thiophene
3-nitro-4-hydroxy-thiophene
3-hydroxy-2-nitro-5-methyl-thiophene
3-hydroxy-2-nitro-4-methyl-thiophene
2-hydroxy-3-nitro-5-phenyl-thiophene
2-nitro-3-hydroxy-4-phenyl-thiophene
2-hydroxy-3-nitro-4-chlorothiophene
2-hydroxy-3-nitro-4-fluorothiophene
and the like.

It is obvious that suitable replacement compounds for 2 with other halo alkyl esters, known in the art, and that suitable replacement of 6 with other diesters, available in the art, will yield all of the ether-amide derivatives within the scope of the claims.

Representative examples of 2 useful in the invention process include, but are not limited to:
Br-CH₂-COOMe,
Cl-CH₂CH₂CH₂COOCH(CH₃)₃,
Br-CH₂CH₂CH₂CH₂COOMe,
Br-CH₂CH₂CH₂CH₂CH₂COOEt,
Br-CH₂CH₂CH₂CH₂CH₂CH₂COOCH₂CH₂CH₂CH₃,
Br-CH₂CH(CH₃)COOMe,
Br-CH₂CH(CH₃)CH₂COOEt,
Br-CH₂CH₂CH₂COOMe,
Br-CH₂CH(OCH₃)CH₂COOCH(CH₃)₂,
Cl-CH₂CH(OCH₂CH₂)CH₂COOMe,
Br-CH₂CH(F)CH₂COOMe,
and the like.

Representative examples of other compounds substitutable for 6 and useful in the invention are:
HOOC(CH₂)₆COOMe,
HOOC(CH₂)₇COOMe,
HOOC(CH₂)₈COOMe,
HOOC(CH₂)₉COOMe,
HOOC(CH₂)₁₀COOMe,
HOOC(CH₂)₁₁COOMe,
HOOC(CH₂)₁₂COOEt,
HOOC(CH₂)₁₃COOCH(CH₃)₂,
HOOC(CH₂)₁₄COOCHCH₂CH₃,
HOOC(CH₂)₁₄COO(CH₂)₃CH₃,
HOOC(CH₂)₁₆COOCH₃,
HOOC(CH₂)₁₇COOCH₃,
HOOC(CH₂)₁₈COOMe,
HOOC(CH₂)₁₉COOEt,
HOOC(CH₂)₂₀COOPh,
HOOC(CH₂)₁₀COOCH₂Ph,
HOOCCH(CH₃)-(CH₂)₁₀COOMe,
HOOC-CH₂CH-(CH₃)(CH₂)₁₀COOMe,
HOOC-CH₂CH₃CH(CH₃)CH₂COOEt,

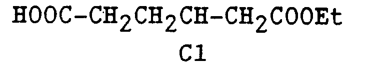

HOOC-CH₂CH(OCH₃)(CH₂)₇COOCH(CH₃)₂,
where Ph is phenyl,
and the like.

Representative examples of compounds produced by this process include those in the following list.

The nomenclature used herein for the acid radicals is:
P(O)(OH)₂, phosphono;
-COOH, carboxy;
-CONH₂, aminocarbonyl;
-SO₃H, sulfo;
-SO₂H, sulfino;
-SSO₃H, thiosulfato, as the sodium salt.
4-(2-(20-Carboxyeicosanoylamino)phenoxy)butyric acid;
4-(2-(19-Carboxynonadecanoylamino)phenoxy)butyric acid;
4-(2-(18-Carboxyoctadecanoylamino)phenoxy)butyric acid;
4-(2-(17-Carboxyheptadecanoylamino)phenoxy)butyric acid;
4-(2-(16-Carboxyhexadecanoyl-N-methylamino)phenoxy)butyric acid;
4-(2-(15-Carboxypentadecanoylamino)phenoxy)butyramide;
4-(2-(14-Carboxytetradecanoylamino)phenoxy)butyric acid;
4-(2-(13-Carboxyl-tridecanoylamino)phenoxy)butyric acid;
4-(2-(12-Carboxy-dodecanoylamino)phenoxy)butyric acid;
4-(2-(11-Carboxy-undecanoylamino)phenoxy)butyric acid;
4-(2-(10-Carboxy-decanoylamino)phenoxy)butyric acid;
4-(2-(9-Carboxy-nonanoylamino)phenoxy)butyric acid;
4-(2-(8-Carboxyoctanoylamino)phenoxy)butyric acid;
4-(2-(7-Carboxyheptanoylamino)phenoxy)butyric acid;
4-(2-(6-Carboxyhexanoylamino)butyric acid;
4-(2-(20-Carboxyeicosanoylamino)phenylthio)butyric acid;
4-(2-(19-Carboxynonadecanoylamino)phenylthio)butyric acid;
4-(2-(18-Carboxyoctadecanoylamino)phenylthio)butyric acid;
4-(2-(17-Carboxyheptadecanoyl-N-ethylamino)phenylthio )butyric acid;
4-(2-(16-Carboxyhexadecanoylamino)phenylthio)butyric acid;
4-(2-(15-Carboxypentadecanoylamino)phenylthio)butyric acid;
4-(2-(14-Carboxytetradecanoylamino)phenylthio)butyramide;
4-(2-(13-Carboxytridecanoylamino)phenylthio acid;
4-(2-(12-Carboxy-dodecanoylamino)phenylthio)butyric acid;
4-(2-(11-Carboxy-undecanoylamino)phenylthio)butyric acid;
4-(2-(10-Carboxydecanoylamino)phenylthio)butyric acid;
4-(2-(9-Carboxynonanoylamino)phenylthio)butyric acid;
4-(2-(8-Carboxyoctanoylamino)phenylthio)butyric acid;
4-(2-(7-Carboxyheptanoylamino)phenylthio)butyric acid;
4-(2-(6-Carboxyhexanoylamino)phenylthio)butyric acid;
3-(2-(16-Carboxyhexadecanoylamino)phenoxy)propionic acid;
4-(2-(15-Carboxyisohexadecanoylamino)phenoxy)butyric acid;
4-(2-(14-Carboxytetradecanoylamino)phenoxy)butyric acid;
5-(2-(13-Carboxytridecanoylamino)phenoxy)valeric acid;
5-(2-(12-Carboxydodecanoylamino)phenoxy)valeric acid;
5-(2-(11-Carboxyisododecanoylamino)valeric acid;
4-(2-(11-Carboxyundecanoylamino)isovaleric acid;
4-(2-(10-Carboxydecanoylamino)isovaleric acid;
5-(2-(9-Carboxynonanoylamino)phenoxy)valeric acid;
6-(2-(9-Carboxynonanoylamino)phenoxy)caproic acid;
6-(2-(8-Carboxyoctanoylamino)phenoxy)caproic acid;
6-(2-(7-Carboxyisooctanoylamino)phenoxy)caproic acid;
7-(2-(7-Carboxyheptanoylamino)phenoxy)enanthic acid;
7-(2-(6-Carboxyhexanoylamino)phenoxy)enanthic acid;
7-(2-(5-Carboxyisohexanoylamino)phenoxy)enanthic acid;
2-(2-(12-Carboxydodecanoylamino)phenoxy)acetic acid;
2-(2-(11-Carboxyundecanoylamino)phenoxy)acetic acid;
2-(2-(10-Carboxydecanoylamino)phenoxy)acetic acid;
3-(2-(9-Carboxynonanoylamino)phenoxy)propionic acid;
3-(2-(12-Carboxydodecanoylamino)phenylthio)propionic acid;
3-(2-(11-Carboxyundecanoylamino)phenylthio)propionic acid;
3-(2-(11-Carboxyundecanoylamino)phenylthio)isobutyric acid;
5-(2-(11-Carboxyundecanoylamino)phenylthio)valeric acid;
5-(2-(10-Carboxydecanoylamino)phenylthio)valeric acid;
5-(2-(9-Carboxynonanoylamino)phenylthio)valeric acid;
4-(2-(12-Carboxydodecanoylamino)phenylthio)isovaleric acid;
4-(2-(11-Carboxydecanoylamino)phenylthio)isovaleric acid;
4-(2-(10-Carboxydecanoylamino)phenylthio)isovaleric acid;
6-(2-(9-Carboxynonanoylamino)phenoxy)caproic acid;
6-(2-(12-Carboxydodecanoylamino)phenylthio)caproic acid;
6-(2-(11-Carboxyundecanoylamino)phenylthio)caproic acid;
7-(2-(11-Carboxyundecanoylamino)-3-methylphenylthio)enanthic acid;
7-(2-(11-Carboxyundecanoylamino)-4-methylphenylthio)enanthic acid;
7-(2-(12-Carboxydodecanoylamino)phenoxy)enanthic acid;
4-(2-(11-Carboxyundecanoylamino)4-methyl-phenoxy) butyric acid;
4-(2-(10-Carboxydecanoylamino)3-methylphenoxy)butyric acid;
4-(2-(9-Carboxynonanoylamino)5-methylphenoxy)butyric acid;
4-(2-(12-Carboxydodecanoylamino)6-methylphenoxy) butyric acid;
4-(2-(11-Carboxydecanoylamino)3-chloro)phenylthio)butyric acid;
4-(2-(10-Carboxydecanoylamino)4-methylphenoxy)butyric acid;
4-(2-(9-Carboxynonanoylamino)5-fluoromethyl)phenylthio)butyric acid;
4-(2-(12-Carboxydodecanoylamino)6-methylphenoxy)butyric acid;
5-(2-(11-Carboxyundecanoylamino)-3-methylthio)phenoxy)valeric acid;
4-(2-(11-Carboxyundecanoylamino)-3-methylsulfonylphenoxy)butyric acid;
4-(2-(11-Carboxyundecanoylamino)-4-methylsulfonyl)phenylthio)butyric acid;
4-(2-(12-Carboxydodecanoylamino)5-ethyl-phenoxy)butyric acid;
4-(2-(11-Carboxyundecanoylamino)4-phenylphenoxy)butyric acid;
4-(2-(10-Carboxydecanoylamino)-3,5-dimethylphenoxy)butyric acid;
4-(2-(9-Carboxynonanoylamino)-4-fluoro-phenoxy)butyric acid;
4-(2-(12-Carboxydodecanoylamino)-5-trifluromethylphenoxy)butyric acid;
4-(2-(11-Carboxyundecanoylamino)5-hydroxy)phenylthio) butyric acid;
4-(2-(10-Carboxydecanoylamino)-4-hydroxy)phenylthio)butyric acid;
4-(2-(9-Carboxynonanoylamino)-3,5-dimethoxyphenylthio)butyric acid;
4-(2-(12-Carboxydodecanoylamino)-5-nitrophenoxy)butyric acid;
5-(2-(11-Carboxyundecanoylamino)-4-nitrophenoxy)valeric acid;
4-(2-(11-Carboxyundecanoylamino)-5-amino-3-methylphenoxy)butyric acid;
4-(2-(11-Carboxyundecanoylamino)-5-amino-4-methylphenylthio)butyric acid;
4-(2-(12-Carboxydodecanoylamino)-4-dimethylaminophenoxy)butyric acid;
4-(2-(11-Carboxyundecanoylamino)-5-ethylaminophenoxy)butyric acid;
3-(2-(12-Carboxydodecanoylamino)phenoxy)-3-methylpropionic acid;
3-(2-(11-Carboxydecanoylamino)phenylthio)-2-chloropropionic acid;
4-(2-(10-Carboxydecanoylamino)phenylthio)-3-methoxybutyric acid;
4-(2-(9-Carboxynonanoylamino)phenylthio)-3-ethoxybutyric acid;
4-(2-(12-Carboxydodecanoylamino)-4-methyl-3-pyridyloxy)butyric acid;
4-(2-(11-Carboxyundecanoylamino)-4-methyl-3-pyridyloxy)butyric acid;
4-(2-(10-Carboxydecanoylamino)-5-methyl-3-pyridyloxy)butyric acid;
4-(2-(9-Carboxynonanoylamino)5-hydroxy-3-pyridyloxy)butyric acid;
4-(2-(12-Carboxydodecanoylamino)-6(dimethylamino)-3-pyridyloxy)butyric acid;
4-(2-(11-Carboxydecanoylamino)-3-pyridylthio)-butyric acid;
4-(2-(10-Carboxydecanoylamino)-6-methylsulfonyl-3-pyridyloxy)butyric acid;
4-(2-(9-Carboxynonanoylamino)5-chloro-3-pyridylthio)butyric acid;
4-(2-(12-Carboxydodecanoylamino)-5-methylpyridylthio)butyric acid;
4-(2-(11-Carboxyundecanoylamino)5-methylsulfonyl-3-pyridylthio)butyric acid;
4-(2-(11-Carboxyundecanoylamino)-6-methyl-3-pyridylthio )butyric acid;
4-(2-(11-Carboxyundecanoylamino)-4,6-dimethyl-3-pyridyloxy)butyric acid;
4-(2-(12-Carboxydodecanoylamino)-5-methylthio-3-pyridyloxy)butyric acid;
4-(2-(11-Carboxyundecanoylamino)pyridyloxy)butyric acid;
4-(2-(10-Carboxydecanoylamino)-5-methoxy-3-pyridyloxy) butyric acid;
4-(2-(9-Carboxynonanoylamino)-4-fluoro-6-methyl-3-pyridyloxy)butyric acid;
4-(2-(12-Carboxydodecanoylamino)5-methylamino-3-pyridyloxy)butyric acid;
4-(2-(11-Carboxyundecanoylamino)4-phenyl-3-pyridylthio )butyric acid;
4-(2-(10-Carboxydecanoylamino)5-methyl-3-pyridylthio)butyric acid;
4-(2-(9-Carboxylnonanoylamino)6-methoxy-3-pyridylthio) butyric acid;
4-(2-(12-Carboxydodecanoylamino)-6-trifluoromethyl-3-pyridyloxy)butyric acid;
5-(2-(11-Carboxyundecanoylamino)-4-methyl-3-thienyloxy)valeric acid;
4-(2-(11-Carboxyundecanoylamino)-4-methyl-3-thienyloxy)butyric acid;
4-(2-(11-Carboxyundecanoylamino)-4-methyl)-3-thienylthio)butyric acid;
4-(2-(12-Carboxydodecanoylamino)-5-methyl-3-thienylthio)butyric acid;
4-(2-(11-Carboxyundecanoylamino)-4-methyl-3-thienylthio)butyric acid;
4-(2-(10-Carboxydecanoylamino)-5-methyl-3-thienylthio)butyric acid;
4-(2-(9-Carboxynonanoylamino)-4-hydroxy-3-thienyloxy)butyric acid;
4-(2-(12-Carboxydecanoylamino)-4-methylthio-3-thienyloxy)butyric acid;
4-(2-(11-Carboxydecanoylamino)-4-methylthio-3-thienyloxy)butyric acid;
4-(2-(10-Carboxydecanoylamino)-4-methylsulfonyl-3-thienyloxy)butyric acid;
4-(2-(9-Carboxynonanoylamino)-4-methylsulfonyl-3-thienyloxy)butryic acid;
4-(2-(12-Carboxydodecanoylamino)-5-trifluoromethyl-3-thienyloxy)butyric acid;
5-(2-(11-Carboxyundecanoylamino)-5-chloro-3-thienyloxy)valeric acid;
4-(2-(11-Carboxyundecanoylamino)-4-methyl-5-phenyl-3-thienyloxy)butyric acid;
4-(2-(11-Carboxyundecanoylamino)-5-methylamino-3-thienyloxy)butyric acid;
4-(2-(12-Carboxydodecanoylamino)-5-dimethylamino-3-thienyloxy)butyric acid;
4-(2-(11-Carboxyundecanoylamino)-3-thienyloxy)butyric acid;
4-(2-(20-Phosphonoeicosanoylamino)phenoxy)butyric acid;
4-(2-(19-Phosphonononadecanoylamino)phenoxy)butyric acid;
4-(2-(17-Sulfoheptadecanoylamino)phenoxy)butyric acid;
4-(2-(16-Sulfinohexadecanoyl-N-methylamino)phenoxy)butyric acid;
4-(2-(15-Thiosulfatopentadecanoylamino)phenoxy) butyramide sodium salt;
4-(2-(14-Phosphonotetradecanoyl-N-methylamino)phenoxy) butyric acid;
4-(2-(12-Sulfododecanoylamino)phenoxy)butyric acid;
4-(2-(11-Sulfoundecanoylamino)phenoxy)butyric acid;
4-(2-(10-Sulfinodecanoylamino)phenoxy)butyric acid;
4-(2-(9-Thiosulfatononanoylamino)phenoxy)butyric acid, sodium salt;
4-(2-(8-Thiosulfatoctanoylamino)phenoxy)butyric acid, sodium salt;
4-(2-(7-Sulfinoheptanoylamino)phenoxy)butyric acid;
4-(2-(6-Phosphonohexanoylamino)butyric acid;
4-(2-(19-Sulfononadecanoylamino)phenylthio)butyric acid;
4-(2-(18-Sulfinooctadecanoylamino)phenylthio)butyric acid;
4-(2-(17-Thiosulfatoheptadecanoyl N-ethylamino)phenylthio)butyric acid;
4-(2-(16-Phosphonohexadecanoylamino)phenylthio) butyric acid;
4-(2-(14-Sulfotetradecanoylamino)phenylthio)butyric acid;
4-(2-(13-Sulfinotridecanoylamino)butyric acid;
4-(2-(12-Thiosulfatododecanoylamino)phenylthio)butyric acid, salt sodium;
4-(2-(11-Phosphonoundecanoylamino)phenylthio)butyric acid;
4-(2-(10-Sulfinodecanoylamino)phenylthio)butyric acid;
4-(2-(9-Carboxynonanoylamino)phenylthio)butanesulfonic acid;
4-(2-(8-Carboxyoctanoylamino)phenylthio)butanesulfinic acid;
4-(2-(7-Carboxyheptanoylamino)phenylthio)butanethiosulfonic acid;
4-(2-(20-Carboxyeicosanoylamino)thio)phenoxy)butanephosphonic acid;
4-(2-(19-Carboxynonadecanoylamino)phenoxy)butanesulfonic acid;
4-(2-(18-Carboxyoctadecanoyl-N-butylamino)phenoxy)butane-sulfinic acid;
4-(2-(17-Carboxyheptadecanoylamino)phenoxy)butanethiosulfonic acid;
4-(2-(15-Carboxypentadecanoylamino)phenoxy)butanephosphonic acid;
4-(2-(14-Carboxytetradecanoylamino)phenoxy)butanesulfonic acid;
4-(2-(13-n-Carboxytridecanoylamino)phenoxy)butanesulfinic acid;
4-(2-(12-Carboxydodecanoylamino)phenoxy)butanethiosulfonic acid, sodium salt;
4-(2-(10-Carboxydecanoylamino)phenoxy)butanephosphonic acid;
4-(2-(9-Carboxynonanoylamino)phenoxy)butanesulfonic acid;
4-(2-(8-Carboxyoctanoylamino)phenoxy)butanesulfinic acid;
4-(2-(7-Carboxyheptanoylamino)phenoxy)butanethiosulfonic acid, sodium salt;
4-(2-(20-Carboxyeicosanoylamino)phenylthio)butanephosphonic acid;
4-(2-(19-Carboxynonadecanoyl-N-methylamino)phenylthio )butanesulfonic acid;
4-(2-(18-Carboxyoctadecanoylamino)phenylthio)butanesulfonic acid;
4-(2-(17-Carboxyheptadecanoylamino)phenylthio)butanesulfinic acid;
4-(2-(16-Carboxyhexadecanoylamino)phenylthio)butanethiosulfonic acid, sodium salt;
4-(2-(14-Carboxytetradecanoyl N-propylamino)phenylthio)butanephosphonic acid;
4-(2-(13-Carboxytridecanoylamino)phenylthio)butanesulfonic acid;
4-(2-(12-Carboxydodecanoylamino)phenylthio)butanesulfinic acid;
4-(2-(11-Carboxyundecanoylamino)phenylthio)butanethiosulfonic acid, sodium salt;
4-(2-(9-Carboxynonanoylamino)phenylthio)butanephosphonic acid;
4-(2-(8-Carboxyoctanoylamino)phenylthio)butanesulfonic acid;
4-(2-(7-Carboxyheptanoylamino)phenylthio)butanesulfinic acid;
4-(2-(6-Carboxyhexanoylamino)phenylthio)butanethiosulfonic acid, sodium salt;
3-(2-(15-Carboxyisohexadecanoylamino)phenoxy) isobutanephosphonic acid;
3-(2-(14-Carboxytetradecanoylamino)phenoxy)isobutanoic acid;
5-(2-(13-Carboxytridecanoylamino)phenoxy)pentanesulfinic acid;
5-(2-(12-Phosphonododecanoylamino)phenoxy)valeramide;
5-(2-(11-Sulfoundecanoylamino)valeric acid;
5-(2-(10-Sulfinodecanoyl N-methylamino)phenoxy valeric acid;
5-(2-(9-Thiosulfatononanoylamino)phenoxy)valeric acid, sodium salt;
6-(2-(9-Phosphonononanoylamino)phenoxy)caproic acid;
6-(2-(7-Sulfoisooctanoyl-N-methylamino)phenoxy) caproic acid;
7-(2-(7-Sulfinoheptanoyl-N-ethylamino)phenoxy) enanthic acid;
7-(2-(6-Thiosulfatohexanoylamino)phenoxy)enanthamide, sodium salt
7-(2-(5-Phosphonoisohexanoylamino)phenoxy)enanthic acid;
2-(2-(11-Sulfoundecanoylamino)phenoxy)acetic acid;
2-(2-(10-Sulfodecanoyl-N-propylamino)phenoxy)acetic acid;
3-(2-(9-Sulfinononanoylamino)phenoxy)propionic acid;
3-(2-(12-Thiosulfatododecanoylamino)phenylthio) propionamide, sodium salt;
3-(2-(11-Phosphonoundecanoylamino)phenylthio) propionic acid;
3-(2-(11-Sulfoundecanoylamino)-4-methyl-phenylthio) isobutyric acid;
3-(2-(12-Sulfinododecanoylamino)phenylthio) isobutyramide;
5-(2-(11-Thiosulfoundecanoyl-N-butylamino)phenylthio) valeric acid, sodium salt;
5-(2-(10-Phosphonodecanoylamino)phenylthio)valeric acid;
5-(2-(12-Phosphonododecanoylamino)phenylthio) pentane-sulfonic acid;
5-(2-(11-Carboxydecanoylamino)phenylthio) pentane sulfinic acid;
5-(2-(10-Phosphonodecanoylamino)phenylthio)pentanethiosulfonic acid;
6-(2-(12-Phosphonododecanoylamino)phenylthio)hexanephosphonic acid;
4-(2-(11-Sulfinoundecanoylamino)4-methyl-phenoxy) butane-thiosulfonic acid, sodium salt;
4-(2-(12-Sulfododecanoylamino)6-methylphenoxy)butane sulfonic acid;
4-(2-(11-Sulfodecanoylamino)3-chloro)phenylthio)butane-sulfinic acid;
4-(2-(10-Sulfodecanoylamino)4-methylphenoxy)butanethiosulfonic acid, sodium salt;
4-(2-(12-Sulfododecanoylamino)6-methylphenoxy)butane-phosphonic acid;
5-(2-(11-Sulfinoundecanoylamino)-3-methylphenylthio )pentane-sulfonic acid;
4-(2-(11-Sulfinoundecanoylamino)-3-methylsulfonylphenoxy)butane-sulfinic acid;
4-(2-(11-Sulfinoundecanoylamino)-4-methylsulfonyl)phenylthio)butanesulfonic acid;
4-(2-(12-Sulfinododecanoylamino)5-ethyl-phenoxy)butane-phosphonic acid;
4-(2-(10-Sulfinodecanoylamino)-3,5-dimethylphenoxy)butane-sulfonic acid;
4-(2-(9-Thiosulfatononanoylamino)-4-fluoro-phenoxy) butane-sulfinic acid, sodium salt;
4-(2-(12-Thiosulfatododecanoylamino)-5-trifluromethylphenoxy )butane-thiosulfonic acid, sodium salt;
4-(2-(10-Thiosulfatodecanoylamino)-4-hydroxyphenylthio)butane-phosphonic acid, sodium salt;
4-(2-(9-Phosphonononanoylamino)-3,5-dimethoxyphenylthio)butyric acid;
5-(2-(11-Sulfoundecanoylamino)-4-nitrophenoxy)valeric acid;
4-(2-(11-Sulfinoundecanoylamino)-5-amino-3-methylphenoxy )butyric acid;
4-(2-(11-Thiosulfatoundecanoylamino)-5-amino-4-methylphenylthio)butyric acid, sodium salt;
4-(2-(12-Phosphonododecanoylamino)-4-dimethyl-aminophenoxy)butyric acid;
4-(2-(10-Sulfodecanoylamino)-phenoxy)butyric acid;
3-(2-(9-Sulfinononanoylamino)phenoxy)propionic acid;
3-(2-(12-Thiosulfatododecanoylamino)phenoxy)-3-methylpropionic acid, sodium salt;
3-(2-(11-Phosphonodecanoylamino)thienyloxy)-2-chloropropionic acid;
4-(2-(9-Sulfononanoylamino)thienyloxy-3-ethoxybutyric acid;
4-(2-(12-Sulfinododecanoylamino)phenoxy)-2-fluorobutyric acid;
7-(2-(11-Thiosulfatoundecanoylamino)phenoxy)6-aminoenanthic acid, sodium salt;
5-(2-(11-Phosphonoundecanoylamino)-3-methylphenoxy)-4-oxo-valeric acid;
4-(2-12-Sulfododecanoylamino)phenoxy)but-2-enoic acid;
4-(2-(11-Sulfinodecanoylamino)phenoxy)but-2-enoic acid;
4-(2-(10-Thiosulfatodecanoylamino)phenoxy)-4-methylene valeric acid, sodium salt;
4-(2-(9-Phosphonononanoylamino)phenoxy)-4-fluoro-2-butenoic acid;
4-(2-(11-Sulfo-3-methylbutanoylamino)thienyloxy)butyric acid;
4-(2-(4-Sulfino-3-chlorobutanoylamino)thienyloxy)butyric acid;
4-(2-(9-Thiosulfato-2-methoxynonanoylamino)thienyloxy )butyric acid, sodium salt;
4-(2-(4-Phosphono-2-ethoxybutanoylamino)phenoxy) butyric acid;
4-(2-(14-Sulfo-14-fluoro-2-acetamidotetradecanoylamino)-3-methylphenoxy)butyric acid;
4-(2-13-Sulfino-2-oxotridecanoylamino)-4-methylthio)phenyloxy)butyric acid;
4-(2-(12-Thiosulfatododecanoyl-3-en-amino)phenoxy) butyric acid;
4-(2-(11-Phosphonoundecanoyl-7-ene-amino)phenoxy) butyric acid;
4-(2-(4-Sulfo-2-fluoro-2-butenoylamino)phenoxy)butyric acid;
4-(2-(12-Sulfinododecanoylamino)-4-methyl-3-pyridyloxy )butyric acid;
4-(2-(11-Thiosulfatoundecanoylamino)-4-methyl-3-pyridyloxy)butyric acid;
4-(2-(10-Phosphonodecanoylamino)-5-methyl-3-pyridyloxy)butyric acid;
4-(2-(11-Sulfinodecanoylamino)-4-nitro-3-pyridylthiobutyric acid;
4-(2-(10-Thiosulfatodecanoylamino)-6-methylsulfonyl-3-pyridyloxy)butyric acid, sodium salt;
4-(2-(9-Phosphonononanoylamino)5-chloro-3-pyridylthio )butyric acid;
4-(2-(11-Sulfoundecanoylamino)5-methylsulfonyl-3-pyridylthio)butyric acid;
4-(2-(11-Sulfinoundecanoylamino)-6-methyl-3-pyridylthio )butyric acid;
4-(2-(11-Thiosulfatoundecanoylamino)-4,6-dimethyl-3-pyridyloxy)butyric acid, sodium salt;
4-(2-(12-Phosphonododecanoylamino)-5-(methylthio)-3-pyridyloxy)butyric acid;
4-(2-(10-Sulfodecanoylamino)-5-methoxy-3-pyridyloxy) butyric acid;
4-(2-(9-Sulfinononanoylamino)-4-fluoro-6-methyl-3-pyridyloxy)butyric acid;
4-(2-(12-Thiosulfatododecanoylamino)5-(methylamino)-3-pyridyloxy)butyric acid, sodium salt;
4-(2-(11-Phosphonoundecanoylamino)4-phenyl-3-pyridylthio )butyric acid;
4-(2-(9-Sulfounonanoylamino)6-methoxy-3-pyridylthio) butyric acid;
4-(2-(12-Sulfinododecanoylamino)-6-trifluoromethyl-3-pyridyloxy)butyric acid;
5-(2-(11-Thiosulfatoundecanoylamino)-4-methyl-3-thiophenyloxy)valeric acid, sodium salt;
4-(2-(11-Phosphonoundecanoylamino)-4-methyl-3-thiophenyloxy)butyric acid;
4-(2-(12-Sulfododecanoylamino)-5-methyl-3-thiophenylthio)butyric acid;
4-2(-(11-Sulfinoundecanoylamino)-4-methyl-3-thiophenylthio)butyric acid;
4-(2-(10-Thiosulfatodecanoylamino)-5-methyl-3-thienylthio)butyric acid, sodium salt;
4-(2-(9-Phosphonononanoylamino)-4-hydroxy-3-thienyloxy)butyric acid:
4-(2-(11-Sulfodecanoylamino)-4-methylthio-3-thienyloxy)butyric acid;
4-(2-(10-Sulfinodecanoylamino)-4-methylsulfonyl-3-thienyloxy)butyric acid;
4-(2-(9-Thiosulfatononanoylamino)-4-methylsulfonyl-3-thienyloxy)butryic acid, sodium salt;
4-(2-(12-Phosphonododecanoylamino)-5-trifluoromethyl-3-thienyloxy)butyric acid;
4-(2-(11-Sulfoundecanoylamino)-4-methyl-5-phenyl-3-thienyloxy)butyric acid;
4-(2-(11-Sulfinoundecanoylamino)-5-methylamino-3-thienyloxy)butyric acid;
4-(2-(12-Thiosulfatododecanoylamino)-5-dimethylamino-3-thienyloxy)butyric acid, sodium salt;
4-(2-(11-Phosphonoundecanoylamino)-4-amino-3-thienyloxy)butyric acid;

Preferred compounds in the invention include:
4-(2-(11-Carboxyundecanoylamino)phenoxy)butyric acid,
4-(2-(11-Carboxyundecanoylamino)phenylthio)butyric acid,
4-(2-(9-Carboxynonanoylamino)phenoxy)butyric acid,
4-(2-(10-Carboxydecanoylamino)phenoxy)butyric acid,
4-(2-(12-Carboxydodecanoylamino)phenoxy)butyric acid,
4-(2-(13-Carboxytridecanoylamino)phenoxy)butyric acid,
4-(2-(15-Carboxypentadecanoylamino)phenoxy)butyric acid,
4-(2-(11-Carboxyundecanoylamino)-4-methylphenoxy)butyric acid,
4-(2-(11-Carboxyundecanoylamino)-5-methylphenoxy)butyric acid.

The compounds of the present invention, prepared in accordance with the method described above, are, as already described, potent antiandrogens by virtue of their ability to specifically inhibit testosterone-5α-reductase.

Accordingly, the present invention is also particularly concerned with providing a method of treating the hyperandrogenic conditions of acne vulgaris, seborrhea, and female hirsutism by topical administration, and a method of treating all of the above conditions as well as benign prostatic hypertrophy, by oral or parenteral administration, of the novel compounds of the present invention.

The present invention is thus also concerned with providing suitable topical, oral and parenteral pharmaceutical formulations for use in the novel methods of treatment of the present invention.

The compositions containing the compounds of the present invention as the active ingredient for use in the treatment of 5-alpha reductase disorders including benign prostatic hyperplasia can be administered in a wide variety of therapeutic dosage forms in conventional vehicles for. systemic administration, as, for example, by oral administration in the form of tablets, capsules, solutions, or suspensions, of by intravenous injection. The daily dosage of the products may be varied over a wide range varying from 50 to 2,000 mg. The compositions are preferably provided in the form of scored tablets containing 5, 10, 25, 50, 100, 150, 250, and 500 milligrams of the active ingredient for the symptomatic adjustment of the dosage to the patient to be treated. An effective amount of the drug is ordinarily supplied at a dosage level of from about 0.01 mg to about 50 mg/kg of body weight per day. Preferably the range is from about 0.1 mg to 7 mg/kg of body weight per day. These dosages are well below the toxic dose of the product. Capsules containing the product of this invention can be prepared by mixing an active compound of the present invention with lactose and magnesium stearate, calcium stearate, starch, talc, or other carriers, and placing the mixture in gelating capsule. Tablets may be prepared by mixing the active ingredient with conventional tableting ingredients such as calcium phosphate, lactose, corn starch or magnesium stearate. The liquid forms in suitably flavored suspending or dispersing agents such as the synthetic and natural gums, for example, tragacanth, acacia, methylcellulose and the like. Other dispersing agents which may be employed include glycerin and the like. For parenteral administration, sterile suspensions and solutions are desired. Isotonic preparations which generally contain suitable preservative are employed when intravenous administration is desired.

For the treatment of acne vulgaris, seborrhea, female hirsutism, the compounds of the present invention are administered in the formula of pharmaceutical composition comprising the active compound in combination with a pharmacologically acceptable carrier adpated for topical administration. These topical pharmaceutical compositions may be in the form of a cream, ointment, gel or aerosol formulation adapted for application to the skin. These topical pharmaceutical compositions containing the compounds of the present invention ordinarily include about 0.1% to 15%, preferably about 5%, of the active compounds, in admixture with about 95% of vehicle.

The method of preparing the novel compounds of the present invention, already described above in general terms, may be further illustrated by the following examples which should not be construed as being limitations on the scope or spirit of the instant invention.

### EXAMPLE 1

### Preparation of 4-(2-(11-carboxyundecanoylamino)phenoxy)butyric acid

### Step A: Ethyl 4-(2-nitrophenoxy)butyrate (3)

To a stirred solution of 2-nitrophenol (1) (1.4 g, 10 mM) and ethyl 4-bromobutyrate (2.1 g, 1.57 mL, 11 mM) in 35 mL of dry acetone is added 2 g (14.5 mM) of anhydrous, ground potassium carbonate. The resultant colored mixture is then heated under a nitrogen atmosphere at gentle reflux until the color due to the phenol anion has dissipated and a yellow mixture remains. Concentration of the cooled and filtered mixture yields an oil which on flash chromatography (silica gel, ethyl acetate/hexane-or methylene chloride as eluant) yields 2.4 g (96% yield) of the title compound (3) as an oily liquid.

### Step B: Ethyl 4-(2-aminophenoxy)butyrate (4)

A solution of (3) (1.27 g, 5.0 mM) in 15 mL ethyl acetate containing 200 mg of 5% palladium on carbon is reacted in a hydrogen atmosphere (40 psig.) at room temperature until hydrogen uptake ceases. The mixture is then filtered and concentrated in vacuo to yield 1.0+ g of (4) as an oil/low melting solid.

### Step C: Dodecanedioic acid, mono methyl ester (6)

Diethyl dodecanedioate (5) (34.4 g, 0.12 M) is reacted with barium hydroxide octahydrate (19.2 g, 0.06 M) in methanol (240 mL) as per the analogous procedure of Org. Syn., Coll. Vol. III, p. 635 to yield 24.8 g of (6) as a white solid.

### Step D: Dodecanedioic acid, mono methyl ester mono acid chloride (7)

A mixture of mono acid (6) (10.0 g, 0.041 M) and thionyl chloride (12.1 mL, 0.166 M) is refluxed for 5 hours, the excess thionyl chloride removed in vacuo, and the residual acid chloride repeatedly dissolved in dry benzene and concentrated until no thionyl chloride remains to yield 10.8 g of the title compound (7) as a waxy solid.

### Step E: Ethyl 4-(2-(11-carbomethoxyundecanoylamino)phenoxy)butyrate (8)

To a stirred, ice-cold solution of 0.89 g (4.0 mM) amine (4) and dried triethylamine (1.2 mL) in dry ether (40 mL) is added dropwise over ca. 4 minutes a solution of acid chloride (7) (1.04 g, 4.6 mM) in 20 mL of dry ether. The resultant mixture is allowed to stir cold for 20 minutes, and then at room temperature overnight. After filtering off the triethylamine hydrochloride, the ether filtrate is concentrated in vacuo and the residue chromatographed on an 82 g silica gel column using 20% ethyl acetate/ hexane as eluant to give 1.49 g (85%) of (8) as a waxy solid.

The ether/triethylamine in the above reaction may be replaced by methylene chloride/pyridine with similar results. The same compound may also be prepared via direct coupling of acid (6) with the same amine using common coupling reagents, such as dicyclohexylcarbodiimide/N,N-dimethylaminopyridine, and the like.

### Step F: 4-(2-(11-Carboxyundecanoylamino) phenoxy)-butyric acid (9)

To a stirred solution of (8) (1.0 g, 2.22 mM) in methanol (100 mL) is added 1 mL of water followed by dropwise addition of 2.5 N sodium hydroxide solution (4.0 mL). The walls of the reaction flask are rinsed down with 10 mL of methanol and the mixture is stirred under a nitrogen atmosphere until TLC analysis shows no ester (mono- or di-) remaining. The methanol is removed in vacuo, the residue taken up in 100 mL of water, stirred for solution, filtered (20 mL water rinses), and the stirred filtrate acidified dropwise with 2 N hydrochloric acid. Filtration of the resultant precipitate followed by copious water washing and drying gave 0.87 g (96%) of (9) as a chalk-like white solid. The compound was one component by TLC (silica gel, the eluant was 10 mL of 2% methanol in methylene chloride containing 4 drops of glacial acetic acid). mp 128.5-130°C uncorr.
Microanalysis:
- Calc.:: C, 64,84; H, 8.16; N, 3.44.
- Found:: C, 64,90; H, 8.34; N, 3.33.

### Step G: Ethyl 4-(2-Amino-3-methylphenylthio) butyrate (11)

To a stirred deaerated (N₂) solution of 2-aminothiophenol (10) (1.25 g., 10 mM) and ethyl 4-bromobutyrate (2.14 g., 11 mM) in 40 mL. of dry 1,2-dimethoxyethane is added 8.3 g. of solid ground anhydrous potassium carbonate, the resultant mixture deaerated 3X under nitrogen and allowed to stir at room temperature until TLC analysis indicates the reaction is complete. The filtered mixture is then concentrated and the residue flash chromatographed on silica gel (85 g.) using 15% ethyl acetate/hexane as eluant to give 1.8g. of (11) as a pale tan oil.

### Step H: 4-(2-(11-Carboxyundecanoylamino) phenylthio)butyric acid (13)

When amine (11) is acylated with acid chloride (7) as per procedure Step (E), and the resultant diester (ethyl 4-(2-(11-carbomethoxyundecanoylamino)phenylthio)butyrate 12 is hydrolyzed in Step I as per procedure (F), the title compound, 13 m.p. 113.5-115°C is obtained.

The following representative compounds in this series were additionally made by the procedures outlined above:
14) 4-(2-(9-Carboxynonanoylamino)phenoxy)butyric acid, m.p. 121.5-124.5°C.
15) 4-(2-(10-Carboxydecanoylamino)phenoxy)butyric acid, m.p. 110-111.5°C.
16) 4-(2-(12-Carboxydodecanoylamino)phenoxy)butyric acid, m.p. 116-119°C.
17) 4-(2-(13-Carboxytridecanoylamino)phenoxy)butyric acid, m.p. 128-129.5°C.
18) 4-(2-(15-Carboxypentadecanoylamino)phenoxy)butyric acid, m.p. 121-125°C.
19) 5-(2-(11-Carboxyundecanoylamino)phenoxy)valeric acid, m.p. 112-113.5°C.
20) 4-(2-(11-Carboxyundecanoylamino)-3-methylphenoxy) butyric acid, m.p. 134.5-136.5°C.
21) 4-(2-(11-Carboxyundecanoylamino)-4-methylphenoxy) butyric acid, m.p. 99.5-100.5°C.
22) 4-(2-(11-Carboxyundecanoylamino)-5-methylphenoxy) butryic acid, m.p. 109.5-113°C.

### Step J: Benzyl 2-Nitrophenol Ether (23)

When 2-nitrophenol (1)is reacted with benzylbromide under the conditions of Step A the title ether (23) is obtained as a golden oil.

### Step K: Benzyl 2-Aminophenyl Ether (24)

A solution of benzyl 2-nitrophenyl ether (23) (1.15g., 5.0 mM) in ethanol (25 mL) saturated with anhydrous ammonia is stirred under a hydrogen atmosphere (40 p.s.i.) with Raney Nickel (2 g.) until TLC analysis indicates the absence of starting nitro compound. The filtered mixture is freed of excess ammonia by bubbling in anhydrous nitrogen. Removal of ethanol via vacuum distillation at room temperature yields 1.0 g. of title compound (24) as a deep colored crust, which was used as-is in the next reaction. This compound may also be obtained via careful reduction in ethanol or ethyl acetate using palladium on carbon as catalyst, but can be accompanied by slight over-reduction if not monitored.

### Step L: N-Trifluoroacetyl 2-Benzyloxyaniline (25)

To a stirred, near solution of amine (24) (5.0 mM) in dry diethyl ether (30 mL) is added anhydrous sodium carbonate (6.0g., 57 mM) and the resultant mixture cooled in an ice-water bath. Trifluoroacetic anhydride (1.5 mL, 10.6 mM) is added dropwise to this cold mixture over 2 minutes, the color changing to a yellowish red. After 2 hours the cooling-bath is removed and the mixture allowed to stir at ambient temperatures overnight. After filtering, the filtrate is concentrated in vacuo and then pumped to yield 1.3 g. of the title compound (25) as a pale tan (with some reddish-brown color around the edges) crust.

### Step M: N-Methyl-2-Benzyloxyaniline (27)

A well-stirred solution of (25) (0.295 g., 1.0 mM), methyl iodide (0.25 mL, 4.0 mM) and anhydrous acetone (5.0 ml) is set in an oil-bath previously heated to 59°C, and kept for 2 minutes. Powdered anhydrous potassium hydroxide (0.225g., 4.0 mM) is added all at once, and the bath temperature allowed to rise to 65°C. Clumping-up of some of the KOH is observed. After 15 additional minutes, the reaction mixture is removed from the bath, allowed to cool, and the volatiles removed. Methanol (7 mL) is added with stirring to the residue of N-Methyl-N-trifluoroacetyl-2-benzyloxyaniline (26) obtained, followed by water (1 mL), and methanol (2 mL) (to wash down the sides). After stirring overnight at ambient temperatures, the methanol is removed in vacuo, the residue distributed between ether and water, separated, the organic layer washed with additional water, saturated sodium chloride solution, and dried over sodium sulfate. Concentration of the filtered ether solution gives the title compound (27) (0.212 g.) as an oil. NMR, MS, and TLC indicate little, if any, dimethyl compound.

### Step N: N-(11-(Carbomethoxy)undecanoyl)-N-Methyl-2-Benzyloxyaniline (28)

To a stirred ice-cold solution of (27) (0.21 g., 1.0 mM) in dried methylene chloride (10 mL) containing anhydrous pyridine (0.3 mL) is added (7) (0.27 g., 1.03 mM), dissolved in 5 methylene chloride (5 mL), dropwise over 1 minute (some methylene chloride used as a rinse). After stirring cold for 30 minutes, the mixture is allowed to stir at ambient temperature for completion of the reaction. The reaction mixture is washed IX with IN HCL, dried (Na₂SO₄) and filtered. Flash chromatography (silica gel, 20% ethyl acetate/hexane as eluant) of the residue obtained gives the title compound (28) (0.33 g) as a colorless oil.

### Step O: N-(11-(Carbomethoxy)undecanoyl)-N-Methyl-2-Hydroxy aniline (29)

A solution of (28) (0.11 g., 0.25 mM) in methanol (11 mL) containing 10% palladium on carbon (30 mg.) is shaken in a 40 p.s.i. hydrogen atmosphere until no (V) remained (TLC analysis). The filtered solution was then concentrated in vacuo to give the title compound (29), used immediately in step P.

### Step P: Ethyl 4-(2-N-(11-Carbomethoxyundecanoyl)-N-(methyl)-amino)phenoxybutyrate (30)

To a stirred solution of (29) (0.087 g., 0.25 mM) and ethyl 4-bromobutyrate (0.115 mL, 0.80 mM) in anhydrous acetone (10 mL) is added anhydrous ground potassium carbonate (0.45 g., 3.2 mM) and the resultant mixture heated under gentle reflux under a nitrogen atmosphere for 24 hours. The mixture is cooled, filtered, and concentrated, and the residue flash chromatographed (silica gel; 20% ethyl acetate/ hexane eluant) to give 80 mg. of the title compound (30) as a colorless oil.

### Step Q: 4-(2-N-(11-Carboxyundecanoyl)-N-(methyl)-amino)-phenoxybutyric Acid (31)

When (30) (0.055 g., 0.118 mM) is hydrolyzed as per its N-desmethyl analog (Step F, supra), and the resultant oil obtained after acidification is extracted with methylene chloride, there is obtained the title compound (31), (51 mg.), as a colorless oil.

### Step R: Ethyl 4-(2-(11-Bromoundecanoylamino) phenoxy)-butyrate (32)

To a solution of (4) (2.60 g., 11 mM) and 11-bromoundecanoic acid (2.65 g., 10 mM) in anhydrous methylene chloride (90 mL) is added 4-(dimethylamino)pyridine (1.22 g., 10 mM) followed by N,N′-dicyclohexylcarbodiimide (2.3 g., 11 mM) (4X5 mL of methylenechloride rinses). Precipitation of dicyclohexylurea (DCU) begins within 4 minutes. When TLC analysis indicates the reaction is complete, the mixture is filtered, the filtrate concentrated in vacuo and the residue extracted with ether. The combined ether extracts are washed IX with IN hydrochloric acid, IX saturated sodium chloride solution, dried (Na₂SO₄) and concentrated to a residue which is stirred, filtered, and concentrated alternately with ether and methylene chloride until all DCU is removed. Concentration of the final solution yields the title product (32) (2.35 g.) as an oil that goes readily to a wary solid.

Attempted purification of an earlier run via column chromatography (silica gel;20% ethyl acetate/ hexane as eluant) gave an impure product of greatly diminished yield.

### Step S: Diethyl 10-(N-((2-(3-Carboethoxy)propyloxy)phenyl)carboxamido)decylphosphonate (33)

A stirred mixture of (32) (0.235 g., 0.5 mM) and triethyl phosphite (TEP) (0.3 mL) is heated at 180°C (bath temperature) under a nitrogen atmosphere for 8 hours, cooled, excess TEP removed in vacuo, and the residue flash chromatographed (Silica gel; ethyl acetate as eluant) to yield product 33 (0.13 g.) as a clear colorless oil.

### Step T:

Cleavage of the phosphonate ester (33) using bromotrimethylsilane (procedure of J.C.S. Chem. Comm. p.739 (1979) yields 10-(N-((2-(3-(Carboethoxy)propyloxy)phenyl)carboxamido)decylphosphonic acid (34).

### Step U:

Further hydrolysis of (34) using the procedure of Example (F) above yields the corresponding di-acid, 10-(N-((2-(3-Carboxy)propyloxy)phenyl) carboxamido)-decylphosphonic acid (35).

### Step V: 10-(N-((2-(3-Carboethoxy)propyloxy)phenyl)carboxamido)decaneisothiouronium bromide (36)

A stirred solution of (32) (0.047 g., 0.1 mM) in ethanol (2 mL) is reacted with thiourea (0.010 g., 0.13 mM) under the same conditions as (in Step W.). Concentration of the reaction mixture yields the title compound (36) (contaminated with a small amount of thiourea) which slowly solidifies in crystalline circles on standing. Stirring with dried chloroform followed by filtration and concentration yields the product as a thick wax.

### Step W: Sodium 10-(N-((2-(3-Carboethoxy)propyloxy)phenyl)carboxamido)decanethiosulfate (37)

To a stirred solution of (32) (0.047 g., 0.1 mM) in ethanol (2.0 mL) is added water (10 drops, slowly) followed by sodium thiosulfate (0.035 g., 0.14 mM), and the reaction mixture heated in an oil-bath (bath temperature ca. 90°C) under a nitrogen atmosphere until TLC analysis indicated no starting bromo compound. The cooled mixture was then concentrated to remove the ethanol and water yielding a white crust. Extraction of this crust with chloroform, followed by filtration from inorganics, yielded product (37) (49 mg.) as a glaze which goes with time to a waxy solid. This product has appreciable water solubility.

Oxidation of (36) or (37) as per the analogous procedures in J.S. Showell et al., J. Org. Chem 27 (1962) 2853 or C. Ziegler et al J. Org. Chem. (1951) 621) yields the corresponding sulfonic acid (38).

### Step X: Ethyl 4-(2-Nitropyrid-3-yloxy)butyrate (40)

This compound was prepared from ethyl 4-bromobutyrate 2 and 2-nitro-3-pyridinol (39) via the procedure of Step (A), supra. Following flash chromatography (silica gel; 1.5% methanol/methylene chloride eluant) a dilute sodium bicarbonate wash of an ether solution of the product was necessary to remove traces of starting phenol. The product (40) was obtained in 76% yield as a pale yelow oil.

### Step Y: Ethyl 4-(2-Aminopyrid-3-yloxy)butyrate (41)

This compound was prepared via reduction of (40) as per the procedure of step (B), above. The amine (41) was obtained as a waxy solid.

### Step Z: Ethyl 4-(2-(11-Carbomethoxyundecanoylamino) pyrid-3-yloxy)butyrate (42)

When (41) and (7) are reacted as per the procedure of step (E), above, the title compound (42) is obtained. Hydrolysis will yield the corresponding di-acid (4-(2-(11- Carboxyundecanoylamino)pyrid-3-yloxy)butyric acid (43).

### Step AA: N-(11-Carbomethoxyundecanoyl)-2-hydroxyaniline (44)

To a stirred near solution of 2-aminophenol (0.24 g, 2.2 mM) in anhydrous methylene chloride (25 mL) was added dry pyridine (0.66 mL) and the mixture cooled in an ice-water bath. A solution of (7) (0.525 g, 2.0 mM) in methylene chloride (2 mL) was added over 1 minute (2X1.5 mL methylene chloride rinses), and the mixture allowed to stir cold. After 30 minutes the bath was removed. After stirring overnight at ambient temperatures, the mixture was filtered, the solvents removed in vacuo, and the residue pumped to remove all traces of pyridine. The product, (44) was used as is in subsequent steps.

### Step BB: 4-(2-(11-Carbomethoxyundecanoylamino) phenoxy)-butyronitrile (45)

When (44) and 4-bromobutyronitrile are reacted under the conditions of step (A), above, (45) is obtained as a waxy solid. Conversely, reacting (44) with ethyl 4-bromobutyrate as in step (A) yields (8).

### Step CC: 4-(2-(11-Carbomethoxyundecanoylamino) phenoxy)-butyramide (46)

To a stirred solution of (45) (11 mg, 0.027 mM) in methylene chloride (3 mL) is added activated manganese dioxide (100 mg) and the resultant suspension allowed to stir stoppered at room temperature. After a few days some additional methylene chloride and manganese dioxide (100 mg) are added and the reaction allowed to continue. This is repeated one additional time. When TLC analysis shows no nitrile remains the mixture is filtered, the catalyst washed well with fresh methylene chloride, and the filtrate concentrated to yield the title product, (46) as a waxy solid.

The above new isolated, purified compounds had NMR and Mass spectra consistent with their assigned chemical structures. All melting points were taken on an A.O. Spencer hot stage and are uncorrected.

## Claims

1. A compound of the structure: 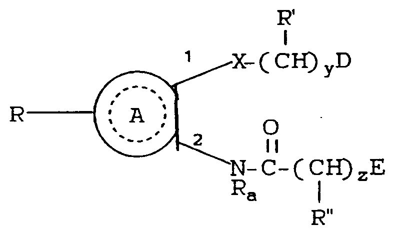 wherein
A is an 1,2-disubstituted aromatic ring selected from
(a) benzene;
(b) 5-6 membered heteroaromatic ring, containing 1-2 N atoms, 1 S or O atom, or combination thereof;
D and E are independently -COOH, -CONH₂, -CONHR_{b}, SO₂OH, -SO₂NH₂, -CO₂R_{b}, -SSO₂ONa, SO₃OH, PH(O)(OH), P(O)(OH)₂;
X is O, S, SO or SO₂:
R is H,
C₁-C₄ alkyl,
phenyl or substituted phenyl,
halo,
haloalkyl,
hydroxy,
carboxy,
cyano,
C₁-C₄ alkoxy,
C₁-C₄ alkylthio,
C₁-C₄ alkylsulfinyl,
C₁-C₄ alkylsulfonyl,
amino,
nitro,
C₁-C₄ mono or di-alkylamino;
R′ and R˝ are independently
H,
halo,
C₁-C₄ alkyl or C₁-C₄ alkoxy,
amino, or oxo, where CH-R′ or CH-R˝
in the formula become -C=O;
Rₐ is H, C₁-C₄ alkyl;
R_{b} is C₁-C₁₂ alkyl, phenyl, or phenyl C₁-C₄ alkyl;
y is 1-6;
z is 6-20; and
wherein 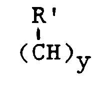 and 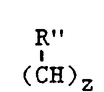 can independently represent substituted or
unsubstituted alkyl or alkenyl radicals containing at least one alkene bond;
and pharmaceutically acceptable salts and esters thereof.

2. The compound of claim 1 wherein X is O.

3. The compound of claim 1 wherein R is H.

4. The compound of claim 1 wherein one of R′ or R˝ is H.

5. The compound of claim 1 wherein y is 3.

6. The compound of claim 1 wherein (Z) is 8-14.

7. The compound of claim 1 wherein both 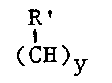 and 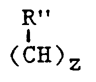 are alkyl.

8. The compound of claim 1 wherein ring A is an 1,2-disubstituted 5-6 membered heteroaromatic ring, containing 1-2 N atoms, 1 S or O atom, or combination thereof.

9. The use of a compound of Claim 1 for the manufacture of a medicament for treating the hyperandrogenic condition of acne vulgaris, seborrhea, female hirsutism, and benign prostatic hyperplasia.

10. A pharmaceutical composition comprising a pharmaceutically acceptable carrier and a therapeutically effective amount of a compound according to Claim 1.
